# EUROPÄISCHE PATENTSCHRIFT

(11) **EP 0 469 411 B1**
(45) Veröffentlichungstag und Bekanntmachung des Hinweises auf die Patenterteilung: **11.06.1997**
(21) Anmeldenummer: 91112154.9
(22) Anmeldetag: 20.07.1991
(51) Int. Cl.: C07D 333/08, C07D 333/12, C07D 333/16, C07D 333/28, C07D 277/22, A01N 43/10, A01N 43/78

(54) **Acetylenderivate, ihre Herstellung und Verwendung zur Bekämpfung von Insekten und Akaridien**
Acetylenic derivatives, their preparation and use to combat insects and acarides
Dérivés acétyléniques, leur préparation et leur utilisation pour combattre des insectes et des acariens

(30) Priorität: 31.07.1990 DE 4024281
(43) Veröffentlichungstag der Anmeldung: 05.02.1992
(73) Patentinhaber: BASF Aktiengesellschaft, 67063 Ludwigshafen (DE)
(72) Erfinder: Rentzea, Costin, Dr., W-6900 Heidelberg (DE); Kardorff, Uwe, Dr., W-6800 Mannheim 1 (DE); Kuenast, Christoph, Dr., W-6701 Otterstadt (DE); Theobald, Hans, Dr., W-6703 Limburgerhof (DE); Kuekenhoener, Thomas, Dr., W-6710 Frankenthal (DE)

(56) Entgegenhaltungen:
- WO-A-88/00467
- US-A- 4 788 207
- US-A- 4 889 867
- CHEMICAL ABSTRACTS, Band 72, Nr. 5, 2. Februar 1970, Seite 321, Zusammenfassung no. 21696h, Columbus, Ohio, US
- JOURNAL OF THE CHEMICAL SOCIETY, PERKIN TRANSACTIONS 1, Nr. 20, 1973, Seiten 2241-2249, Letchworth, GB, J.A. CLAISSE et al.
- CHEMICAL AND PHARMACEUTICAL BULLETIN, Band 35, Nr. 2, 1987, Seiten 823-828, Tokyo, JP, T. SAKAMOTO et al.
- CHEMICAL ABSTRACTS, Band 93, Nr. 7, 18. August 1980, Seite 947, Zusammenfassung Nr. 71446f, Columbus, Ohio, US
- CHEMICAL ABSTRACTS, Band 106, Nr. 21, 25. Mai 1987, Seite 715, Zusammenfassung Nr. 176241a, Columbus, Ohio, US
- CHEMICAL AND PHARMACEUTICAL BULLETIN, Band 29, Nr. 12, 1981, Seiten 3543-3547, Tokyo, JP, H. YAMANAKA et al.
- CHEMICAL AND PHARMACEUTICAL BULLETIN, Band 29, Nr. 12, 1981, Seiten 3548-3553, Tokyo, JP, H. YAMANAKA et al.
- JOURNAL OF HETEROCYCLIC CHEMISTRY, Band 19, Nr. 1982, Seiten 145-151, Provo, US, L. VO-QUANG et al.

## Beschreibung

Die vorliegende Erfindung betrifft Acetylenderivate der allgemeinen Formel I

R¹-C≡C-R² I

in der die Substituenten folgende Bedeutung haben:
- R¹: 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Alkenyl oder C₃-C₈-Cycloalkyl;
- R²: Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio,
wobei R¹ nicht 2-Thienyl bedeutet, wenn R² für Phenyl oder 4-Methylphenyl steht, R¹ nicht 2-Thienyl, 5-Chlor-2-thienyl, 3,5-Dichlor-2-thienyl, 3-Methyl-2-thienyl, 5-Methyl-2-thienyl, 5-Methoxy-2-thienyl, 3-Thienyl-, 2,5-Dichlor-3-thienyl, 2-Methyl-3-thienyl, 5-Chlor-3-thienyl bedeutet, wenn R² für 4-C₁-Halogenalkylphenyl steht, R¹ nicht 2-Thienyl bedeutet, wenn R² für 2-X-4-C₁-Halogenalkylphenyl mit X = Fluor, Chlor, Methyl, Ethyl, i-Propyl, n-Butyl, Trifluormethyl, Methoxy oder Ethoxy steht, R¹ nicht 5-Chlor-2-thienyl bedeutet, wenn R² für 2-X-4-C₁-Halogenalkylphenyl mit X = Fluor, Chlor, Methyl oder Methoxy steht, R¹ nicht 3-Thienyl bedeutet, wenn R² für 2-X-4-C₁-Halogenalkylphenyl mit X = Fluor, Chlor, Methyl, Methoxy oder Trifluormethyl steht, R¹ nicht 3-Methyl-2-thienyl, 2,5-Dichlor-3-thienyl oder 2-Methyl-3-thienyl bedeutet, wenn R² für 2-X-4-C₁-Halogenalkylphenyl mit X = Fluor oder Chlor steht, R¹ nicht 3-Brom-5-methyl-2-thienyl bedeutet, wenn R² für Phenyl steht, R¹ nicht 5-Methyl-2-thienyl bedeutet, wenn R² für Phenyl steht, und R¹ nicht 3-Thienyl bedeutet, wenn R² für Phenyl steht.

Außerdem betrifft die Erfindung Verfahren zur Herstellung dieser Verbindungen und sie enthaltende Insekten- und Akaridienbekämpfungsmittel sowie Verfahren zur Insekten- und Akaridienbekämpfung mit Hilfe von Acetylenderivaten der Formel IA,

R¹-C≡C-R² Ia

in der die Substituenten folgende Bedeutung haben:
- R¹: 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkylthio, C₃-C₆-Alkenyl oder C₃-C₈-Cycloalkyl;
- R²: Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio.

Aus der Literatur sind 1-Phenyl-2-thienylacetylenderivate als Nematizide bekannt [Organometallic Chem., 93, 259 f. (1975); Tetrahedron 40, 2773 f. (1984); Agric. Biol. Chem., 46, 309 f. (1982); JP-A 81/123,903 [Chem. Abstr. 96, 16085 z (1982)]].

Weiter sind in US 4,788,207 und US 4,889,867 1-Phenyl-2-thiazolylacetylenderivate, bei denen die Thiazolylreste ihrerseits durch Thienyl oder Phenyl substituiert sind, als Insektizide und Akarizide beschrieben. Aus Chemical and Pharmaceutical Bulletin, Band 35, Nr. 2, 1987, 824 ist weiter 1-Phenyl-2-thiazolyl-acetylen bekannt. Außerdem werden in WO-A-8 800 467 Thienylacetylenderivate mit einem oder zwei an die acetylenischen Kohlenstoffatome gebundenen Thienylresten, die ihrerseits durch Thienyl oder Phenyl substituiert sind, beschrieben. Aus Chem. Abstr. 72: 21696H ist weiter die Verwendung von 1-(1,2,4-Oxadiazol-3-yl)-2-phenylacetylen und dessen Derivaten zur Bekämpfung von Parasitenbefall bei Tieren bekannt.

Aufgabe der vorliegenden Erfindung waren neue wirksame Insektizide und Akarizide.

Demgemäß wurden die eingangs definierten Acetylenderivate I gefunden. Außerdem wurden Verfahren zur Herstellung dieser Acetylenderivate sie enthaltende Insekten- und Akaridienbekämpfungsmittel und Verfahren zu ihrer Verwendung gefunden.

Die Acetylenderivate I sind auf verschiedenen Wegen zugänglich. Besonders vorteilhaft erhält man sie nach einem der im nachfolgenden beschriebenen Verfahren A und B.

### Verfahren A:

Man erhält die Acetylenderivate der Formel I beispielsweise dadurch, daß man ein Alkin der allgemeinen Formel IIa bzw. IIb, in an sich bekannter Weise in einem inerten organischen Lösungsmittel in Gegenwart einer Base und eines Palladium- und Kupferkatalysators mit einem Arylhalogenid der allgemeinen Formel IIIa bzw. IIIb umsetzt.

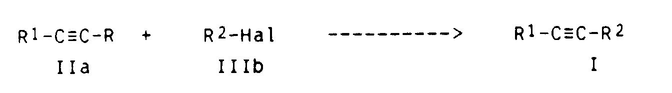

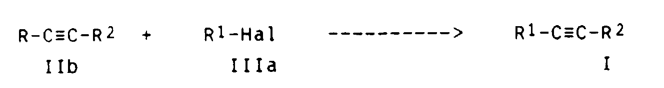

In den Formeln IIa und IIb bedeutet R Wasserstoff oder eine (C₁-C₃-Alkyl)₂C(OH)-Schutzgruppe wie 2-Hydroxypropyl, 2-Hydroxybutyl und 3-Hydroxypentyl.

Hal in den Formeln IIIa und IIIb steht für ein Halogenatom wie Fluor, Chlor, Brom und Jod, insbesondere Brom oder Jod.

Die Umsetzung von IIa, b mit IIIa, b wird im allgemeinen in einem inerten organischen Lösungsmittel, gegebenenfalls in Gegenwart von Wasser und in Gegenwart eines Palladiumkatalysators und eines Kupferkatalysators, einer Base und gegebenenfalls in Gegenwart eines Phasentransferkatalysators bei Temperaturen von -10 bis 150°C, insbesondere 20 bis 130°C durchgeführt.

Die Umsetzung verläuft gewöhnlich oberhalb von 20°C mit ausreichender Geschwindigkeit. 120°C müssen im allgemeinen nicht überschritten werden. Da die Umsetzung in einigen Fällen unter Wärmeentwicklung verläuft, kann es von Vorteil sein, eine Kühlmöglichkeit vorzusehen.

Als Lösungsmittel eignen sich beispielsweise Kohlenwasserstoffe wie Pentan, Hexan, Benzol, Toluol und Xylole; Ether wie Dietyhl-, Di-n-butylether, Methyl-tert.-butylether, Tetrahydrofuran und Dioxan; Ketone wie Aceton und Methylethylketon; Nitrile wie Acetonitril; Alkohole wie Methanol und Ethanol; aprotische Lösungsmittel wie Dimethylformamid, Dimethylsulfoxid und Pyridin, insbesondere Acetonitril, Ethanol, Dimethylformamid und entsprechende Gemische.

Man verwendet normalerweise mindestens äquivalente Mengen einer Base, kann diese aber auch im Überschuß oder gegebenenfalls als Lösungsmittel einsetzen.

Als Basen eignen sich beispielsweise Hydroxide von Alkali und Erdalkalimetallen wie Natriumhydroxid, Kaliumhydroxid und Calciumhydroxid; Alkoholate von Alkali und Erdakalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- oder Erdalkalicarbonate wie Natriumcarbonat, Kaliumcarbonat oder Calciumcarbonat; aliphatische Amine wie Dimethylamin, Triethylamin, Tripropylamin, Tributylamin oder Pyrrolidin; aromatische Amine wie Pyridin, N,N-Dimethylanilin und N,N-Diethylanilin.

Als Katalysatoren werden Palladiumverbindungen wie Palladiumchlorid, Palladiumacetat, Bis(triphenylphosphin)palladiumacetat, Bis(triphenylphosphin)palladiumchlorid, Bis(triphenylphosphin)palladiumbromid, Palladium-bis-(acetonitril)-dichlorid, Palladium-bis(benzonitril)-dichlorid, Palladium-acetylacetonat, Tetrakis-(triphenylphosphin)palladium und Kupferverbindungen wie Kupferiodid, Kupferbromid, Kupferacetat und Kupferacetylacetonat verwendet.

Als Phasentransfer-Katalysatoren kommen vorzugsweise quaternäre Ammonium- und Phosphoniumsalze wie Tetrabutylammonium-chlorid, -hydrogensulfat, -hydroxid, -bromid oder -iodid, Benzyltriethylammoniumchlorid, Cetyltrimethylammmoniumchlorid, Benzyltriphenylphosphoniumchlorid oder Kronenether wie 12-Krone-4, 15-Krone-5 und 18-Krone-6 in Betracht.

Die Ausgangsstoffe werden üblicherweise in stöchiometrischen Mengen miteinander umgesetzt, zur Steigerung der Ausbeute kann es jedoch vorteilhaft sein, einen der Ausgangsstoffe in einem Überschuß von 0,1 bis 10 mol-Äq., insbesondere 0,2 bis 1,5 mol-Äq. einzusetzen. Die Katalysatoren werden bevorzugt in 0,01 bis 0,2 molaren Mengen eingesetzt.

Die Reaktionsgemische werden in üblicher Weise aufgearbeitet, z. B. durch Versetzen mit Wasser, Trennen der Phasen und Reinigung der Rohprodukte mittels Säulenchromatographie.

### Verfahren B:

Man erhält die Verbindungen der Formel I beispielsweise auch dadurch, daß man ein Dibromethan der allgemeinen Formel IV, in an sich bekannter Weise in einem inerten organischen Lösungsmittel in Gegenwart einer Base umsetzt.

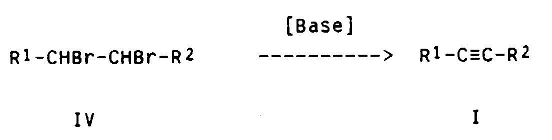

Die Umsetzung erfolgt im allgemeinen bei Temperaturen von -20 bis 110°C, vorzugsweise von 0 bis 90°C.

Als Lösungsmittel eignen sich beispielsweise die vorstehend bei Verfahren A genannten. Insbesondere kommen die folgenden in Betracht: Methanol, Ethanol, Propanol, 2-Propanol und deren Gemische mit Wasser.

Als Basen kommen in diesem Verfahren die vorstehend genannten in Betracht.

Im Hinblick auf die bestimmungsgemäße Verwendung der Verbindungen IA in Insekten- und Akaridienbekämpfungsmitteln kommen als Substituenten folgende Reste in Betracht:
- R¹: 2-Thienyl oder 3-Thienyl, welches ein bis drei der folgenden Reste tragen kann:

Nitro;
Halogen wie Fluor, Chlor, Brom und Jod, insbesondere Fluor;
C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl, Ethyl und 1-Methylethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2,Chlor-2,2-difluorethyl, 2,2-Dichlor-2-fluorethyl, 2,2,2-Trichlorethyl und Pentafluorethyl, insbesondere Trichlormethyl und Trifluormethyl;
C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, Ethoxy und 1-Methylethoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethyloxy und Pentafluorethyloxy;
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-chlor-2,2-difluorethylthio, 2,2-Dichlor-2-fluorethylthio, 2,2,2-Trichlorethylthio und Pentafluorethylthio;
C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 2-Propenyl, 2-Butenyl, 2-Methyl-2-propenyl, 2-Pentenyl, 2-Methyl-2-butenyl, 3-Methyl-2-butenyl und 1,2-Dimethyl-2-propenyl, insbesondere 2-Propenyl, 2-Butenyl und 3-Methyl-2-butenyl;
oder C₃-C₈-Cycloalkyl wie Cyclopropyl, Cyclobutyl, Cyclopentyl, Cyclohexyl, Cycloheptyl, Cyclooctyl, vorzugsweise Cyclopropyl, Cyclopentyl und Cyclohexyl, insbesondere Cyclopropyl;

- R²: ein ein- bis dreikerniges aromatisches Ringsystem wie Phenyl, 1-Naphthyl und 2-Naphthyl, welches ein bis fünf Halogenatome wie Fluor, Chlor, Brom und Jod, insbesondere Fluor und Chlor und/oder ein bis drei der folgenden Gruppen tragen kann: Nitro, Cyano,

C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl und Ethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;
C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy, insbesondere Methoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, difluormethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, fluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
C₁-C₄-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl und 1,1-Dimethylethoxycarbonyl, insbesondere Methoxycarbonyl und Ethoxycarbonyl;
C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 2-Propenyl, 2-Butenyl und 2,3-Dimethyl-2-butenyl;
Phenyl und Phenoxy, wobei die letztgenannten aromatischen Gruppen ihrerseits ein bis fünf Halogenatome wie Fluor, Chlor, Brom und Jod, vorzugsweise Fluor und Chlor, und/oder ein bis drei der folgenden Gruppen tragen können:
C₁-C₄-Alkyl, wie Methyl, Ethyl, Propyl, 1-Methylethyl, Butyl, 1-Methylpropyl, 2-Methylpropyl und 1,1-Dimethylethyl, vorzugsweise Methyl und Ethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;
C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;
   oder
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio,2-Chlor-2,2-difluorethylthio, 2,2-Dichlor-2-fluorethylthio, 2,2,2-Trichlorethylthio und Pentafluorethylthio, vorzugsweise 2,2,2-Trichlorethylthio.

Beispiele für insbesondere bevorzugte Acetylenderivate der allgemeinen Formel IA sind in der folgenden Tabelle aufgeführt.

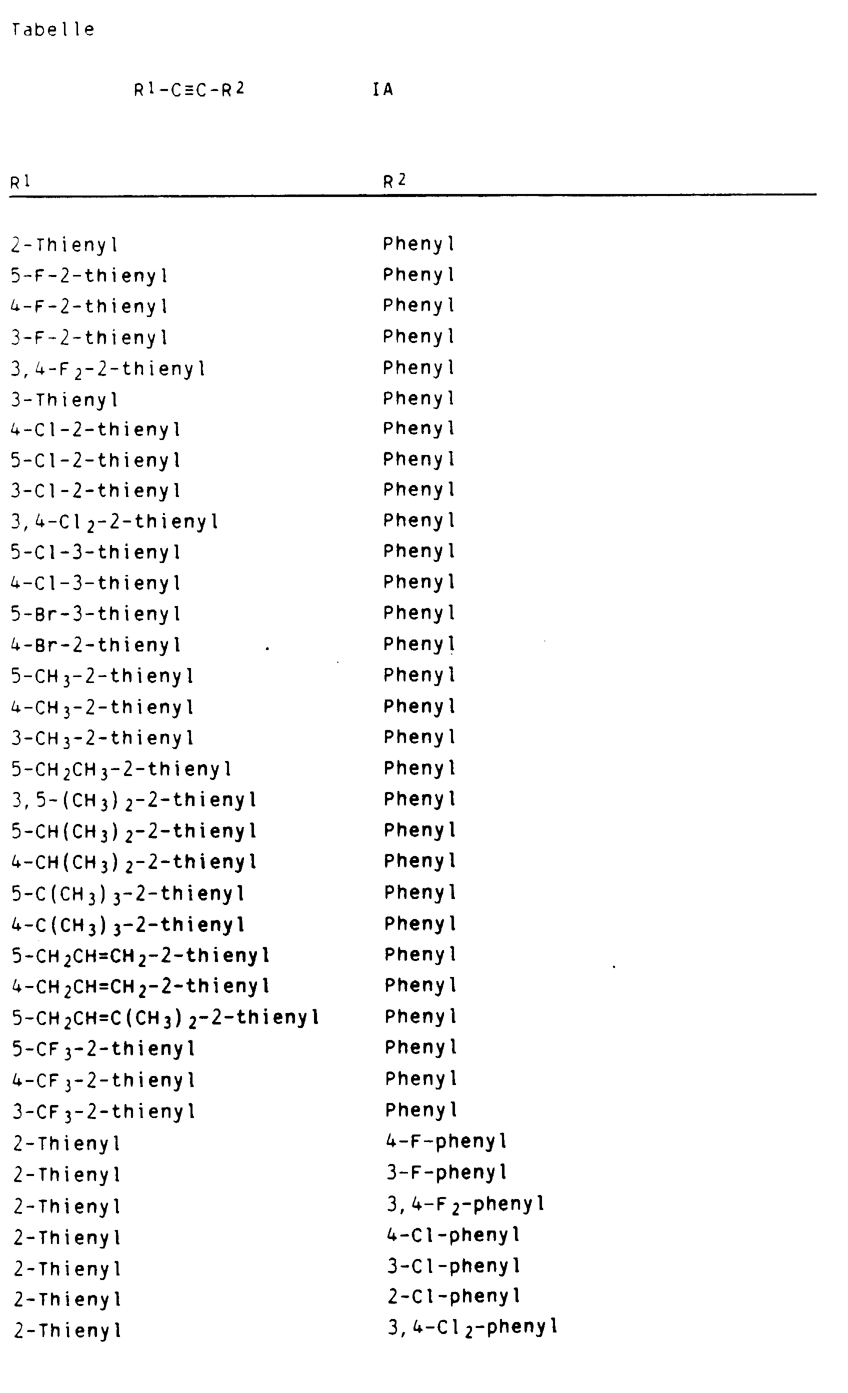

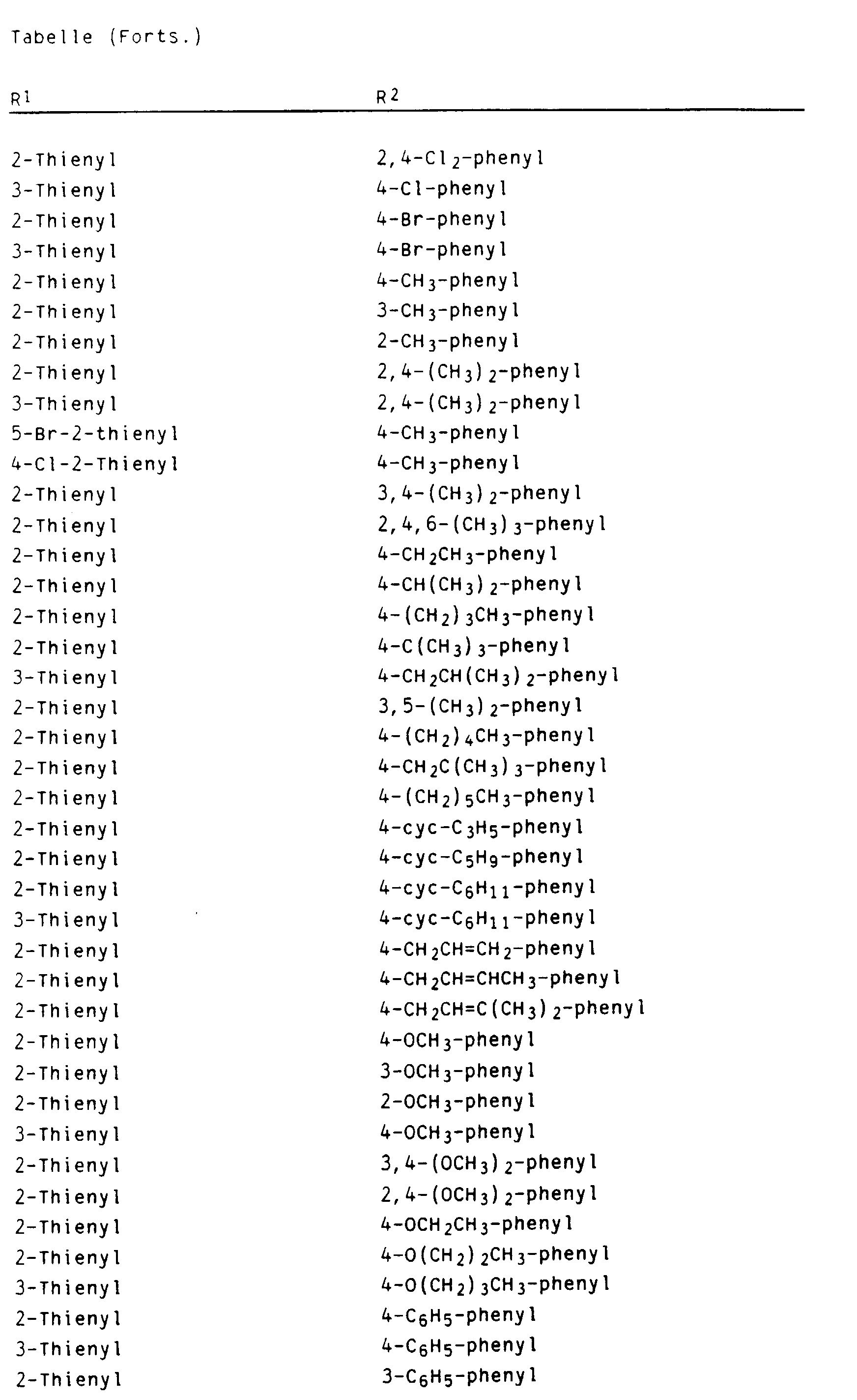

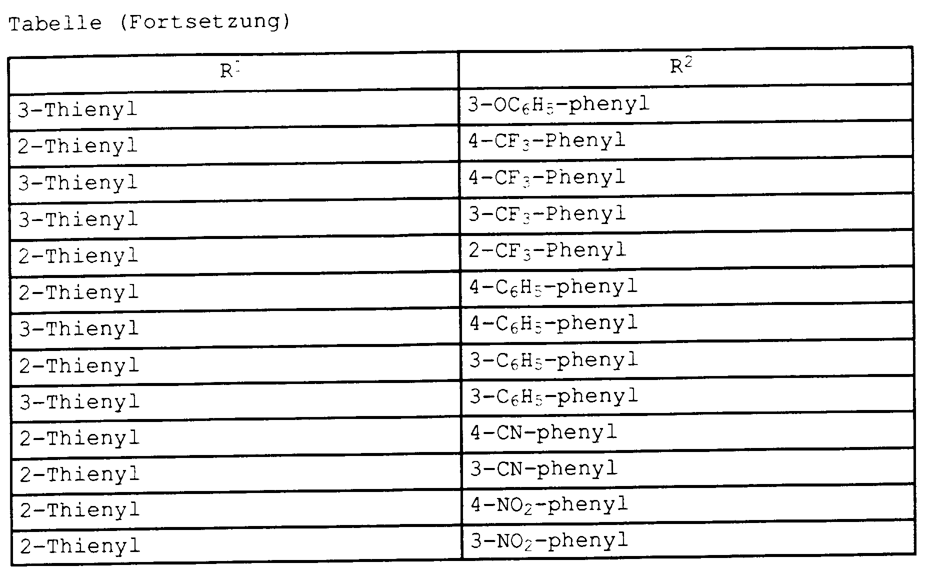

Die Verbindungen Formel IA sind geeignet, Schädlinge aus der Klasse der Insekten, Spinnentiere und Nematoden wirksam zu bekämpfen. Sie können im Pflanzenschutz sowie auf den Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pmonella, Dendrolimus oni, Diaphania nitidalis, Diatraea grandiosella, Earias insulana, Elasmopalpus lignosellus, Eupoecilia ambiguella, Evetria bouliana, Feltia subterranea, Galleria mellonella, Grapholitna funebrana, Grapholitna molesta, Heliothis arminella, Grapholitna funebrana, Grapholitna molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella.

Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis gossypii, Aphis pomi, Aphis sambuci, Brachycaudus cardui, Brevicoryne brassicae, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mali, Schizaphis graminum, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, öldispersionen) durch Zusatz von Wasser bereitet werden. Zur Herstellung von Emulsionen, Pasten oder öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, -Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile der Verbindung Nr. 1.001 werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile der Verbindung Nr. 1.003 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile der Verbindung Nr. 1.003 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile der Verbindung Nr. 1.003 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile der Verbindung Nr. 1.001 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile der Verbindung Nr. 1.003 mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile der Verbindung Nr. 1.001 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile des Wirkstoffs Nr. 1.001 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 5 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die im nachstehenden Synthesebeispiel wiedergegebene Vorschrift wurde unter entsprechender Abwandlung der Ausgangsverbindungen zur Gewinnung weiterer Verbindungen IA benutzt. Die so erhaltenen Verbindungen sind in der nachstehenden Tabelle mit physikalischen Angaben aufgeführt.

### Beispiel 1

### 1-(2-Thienyl)-2-phenylacetylen

Die Lösung von 163 g (1 mol) 2-Bromthiophen und 127,5 g (1,25 mol) Phenylacetylen in 850 ml Triethylamin wurde bei 25°C nacheinander mit 1 g (0,00142 mol) Bis-(triphenylphosphin)-palladiumchlorid, 5 g Kupferiodid (0,0262 mol) und 8,5 g Triphenylphosphin (0,0324 mol) versetzt. Nach zwölfstündigem Nachrühren unter Stickstoff-Atmosphäre bei 80°C wurde das Gemisch filtriert und das Filtrat im Vakuum eingeengt. Der Rückstand wurde mit 600 ml Methylenchlorid versetzt, dreimal mit je 200 ml Wasser gewaschen, über Na₂SO₄ getrocknet, im Vakuum eingeengt und anschließend mit 200 ml n-Pentan versetzt. Die so erhaltenen Kristalle wurden abgesaugt und getrocknet. Man erhielt 106 g (57,6 % der Theorie) 1-(2-Thienyl)-2-phenylacetylen als schwach gelbe Kristalle vom Schmp. 49 - 59^{o}C (Wirkstoffbeispiel 1.001).

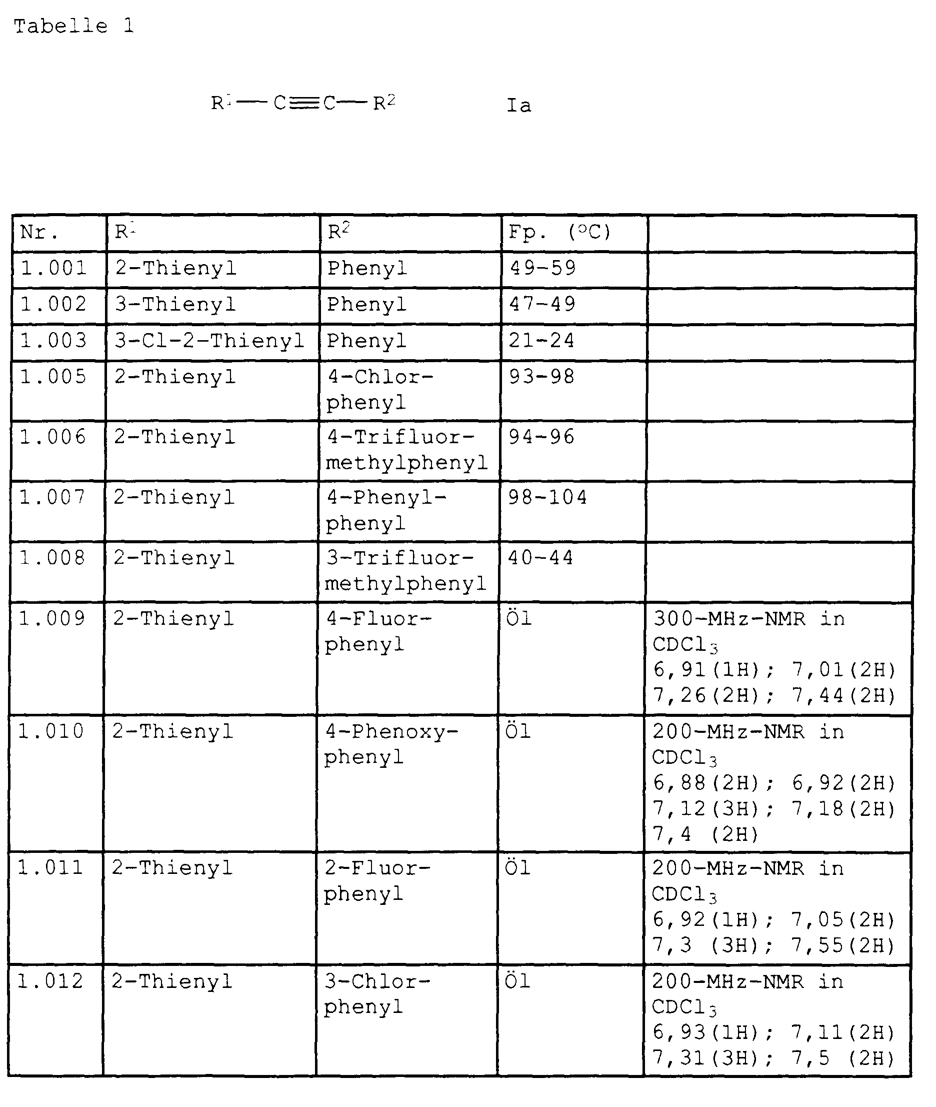

### Anwendungsbeispiele

Die insektizide Wirkung der Verbindungen der allgemeinen Formel IA ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %ige Hemmung bzw. Mortalität hervorriefen (Wirkschwelle bzw. Minimalkonzentration (mg)).

### A. Heliothis virescens, ovo-larvizide Wirkung

Blattstücke von Buschbohnen werden zunächst mit der wässrigen Wirkstoffaufbereitung benetzt und anschließend in einer Petrischale mit ca. 15 Heliothis-Eiern belegt (die Eier sollen nicht älter als 24 h sein).

Nach 4 Tagen wird der Schlupf und die Mortalität von Jungraupen beurteilt.

In diesem Test zeigten die Verbindungen 1.001 und 1.003 Wirkschwellen von 1000 bzw. 400 ppm.

### B. Prodenia litura, Zuchtversuch

Fünf Raupen des Entwicklungsstadiums L3 (10 - 12 mm), die keine feststellbare Schädigung im Kontaktversuch erlitten hatten, werden auf mit dem Wirkstoff benetzten Standardnährboden aufgebracht.

Die Beobachtung erstrekt sich bis zum Schlüpfen der Falter bei einem Kontrollversuch ohne Wirkstoff.

In diesem Test zeigten die Verbindungen 1.001 und 1.003 Wirkschwellen von 1,0 bzw. 0,04 ppm.

## Patentansprüche (Patentansprüche für folgende(n) Vertragsstaat(en): BE, CH, DE, DK, FR, GB, IT, LI, NL)

1. Acetylenderivate der allgemeinen Formel I,
R¹-C≡C-R² I
in der die Substituenten folgende Bedeutung haben:
R¹ 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Alkenyl oder C₃-C₈-Cycloalkyl;
R² Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio,
wobei R¹ nicht 2-Thienyl bedeutet, wenn R² für Phenyl oder 4-Methylphenyl steht, R¹ nicht 2-Thienyl, 5-Chlor-2-thienyl, 3,5-Dichlor-2-thienyl, 3-Methyl-2-thienyl, 5-Methyl-2-thienyl, 5-Methoxy-2-thienyl, 3-Thienyl-, 2,5-Dichlor-3-thienyl, 2-Methyl-3-thienyl, 5-Chlor-3-thienyl bedeutet, wenn R² für 4-C₁-Halogenalkylphenyl steht, R¹ nicht 2-Thienyl bedeutet, wenn R² für 2-X-4-C₁-Halogenalkylphenyl mit X = Fluor, Chlor, Methyl, Ethyl, i-Propyl, n-Butyl, Trifluormethyl, Methoxy oder Ethoxy steht, R¹ nicht 5-Chlor-2-thienyl bedeutet, wenn R² für 2-X-4-C₁-Halogenalkylphenyl mit X = Fluor, Chlor, Methyl oder Methoxy steht, R¹ nicht 3-Thienyl bedeutet, wenn R² für 2-X-4-C₁-Halogenalkylphenyl mit X = Fluor, Chlor, Methyl, Methoxy oder Trifluormethyl steht, R¹ nicht 3-Methyl-2-thienyl, 2,5-Dichlor-3-thienyl oder 2-Methyl-3-thienyl bedeutet, wenn R² für 2-X-4-C₁-Halogenalkylphenyl mit X = Fluor oder Chlor steht, R¹ nicht 3-Brom-5-methyl-2-thienyl bedeutet, wenn R² für Phenyl steht, R¹ nicht 5-Methyl-2-thienyl bedeutet, wenn R² für Phenyl steht, und R¹ nicht 3-Thienyl bedeutet, wenn R² für Phenyl steht.

2. Verfahren zur Herstellung der Verbindungen der Formel I gemäß Anspruch 1, dadurch gekennzeichnet, daß man ein Alkin der allgemeinen Formel IIa bzw. IIb, 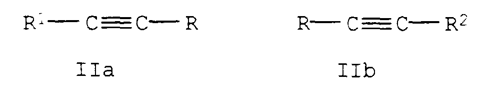 in der R für Wasserstoff oder eine (C₁-C₃-Alkyl)₂C(OH)-Schutzgruppe steht, in an sich bekannter Weise in einem inerten organischen Lösungsmittel in Gegenwart einer Base und eines Palladium- und Kupferkatalysators mit einem Arylhalogenid der allgemeinen Formel IIIa bzw. IIIb, 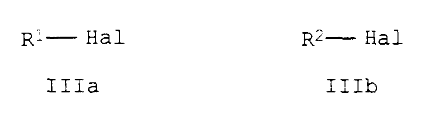 in der Hal für ein Halogenatom steht, umsetzt.

3. Verfahren zur Herstellung der Verbindungen der Formel I gemäß Anspruch 1, dadurch gekennzeichnet, daß man ein Dibromethan der allgemeinen Formel IV, 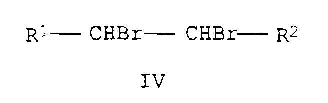 in an sich bekannter Weise in einem inerten organischen Lösungsmittel in Gegenwart einer Base umsetzt.

4. Insekten- und Akaridienbekämpfungsmittel, enthaltend eine wirksame Menge eines Acetylenderivats der allgemeinen Formel I gemäß Anspruch 1 und inerte Zusatzstoffe.

5. Verfahren zur Bekämpfung von Insekten und Akaridien, dadurch gekennzeichnet, daß man die Insekten und Akaridien und/oder ihren Lebensraum mit einer wirksamen Menge eines Acetylenderivats der allgemeinen Formel IA,
R¹-C≡C-R² Ia
in der die Substituenten folgende Bedeutung haben:
R¹ 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkylthio, C₃-C₆-Alkenyl oder C₃-C₈-Cycloalkyl;
R² Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio,
behandelt.

## Patentansprüche (Patentansprüche für folgende(n) Vertragsstaat(en): ES)

1. Insekten- und Akaridienbekämpfungsmittel, enthaltend eine wirksame Menge eines Acetylenderivate der allgemeinen Formel I,
R¹-C≡C-R² I
in der die Substituenten folgende Bedeutung haben:
R¹ 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Alkenyl oder C₃-C₈-Cycloalkyl;
R² Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio,
wobei R¹ nicht 2-Thienyl bedeutet, wenn R² für Phenyl oder 4-Methylphenyl steht, R¹ nicht 2-Thienyl, 5-Chlor-2-thienyl, 3,5-Dichlor-2-thienyl, 3-Methyl-2-thienyl, 5-Methyl-2-thienyl, 5-Methoxy-2-thienyl, 3-Thienyl-, 2,5-Dichlor-3-thienyl, 2-Methyl-3-thienyl, 5-Chlor-3-thienyl bedeutet, wenn R² für 4-C₁-Halogenalkylphenyl steht, R¹ nicht 2-Thienyl bedeutet, wenn R² für 2-X-4-C₁-Halogenalkylphenyl mit X = Fluor, Chlor, Methyl, Ethyl, i-Propyl, n-Butyl, Trifluormethyl, Methoxy oder Ethoxy steht, R¹ nicht 5-Chlor-2-thienyl bedeutet, wenn R² für 2-X-4-C₁-Halogenalkylphenyl mit X = Fluor, Chlor, Methyl oder Methoxy steht, R¹ nicht 3-Thienyl- bedeutet, wenn R² für 2-X-4-C₁-Halogenalkylphenyl mit x = Fluor, Chlor, Methyl, Methoxy oder Trifluormethyl steht, R¹ nicht 3-Methyl-2-thienyl, 2,5-Dichlor-3-thienyl oder 2-Methyl-3-thienyl bedeutet, wenn R² für 2-X-4-C₁-Halogenalkylphenyl mit X = Fluor oder Chlor steht, R¹ nicht 3-Brom-5-methyl-2-thienyl bedeutet, wenn R² für Phenyl steht, R¹ nicht 5-Methyl-2-thienyl bedeutet, wenn R² für Phenyl steht, und R¹ nicht 3-Thienyl bedeutet, wenn R² für Phenyl steht, und inerte Zusatzstoffe.

2. Verfahren zur Herstellung der Verbindungen der Formel I gemäß Anspruch 1, dadurch gekennzeichnet, daß man ein Alkin der allgemeinen Formel IIa bzw. IIb, 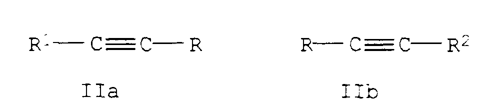 in der R für Wasserstoff oder eine (C₁-C₃-Alkyl)₂C(OH)-Schutzgruppe steht, in an sich bekannter Weise in einem inerten organischen Lösungsmittel in Gegenwart einer Base und eines Palladium- und Kupferkatalysators mit einem Arylhalogenid der allgemeinen Formel IIIa bzw. IIIb, 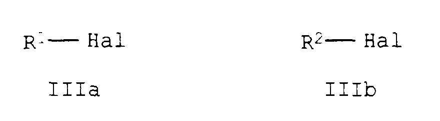 in der Hal für ein Halogenatom steht, umsetzt.

3. Verfahren zur Herstellung der Verbindungen der Formel I gemäß Anspruch 1, dadurch gekennzeichnet, daß man ein Dibromethan der allgemeinen Formel IV, 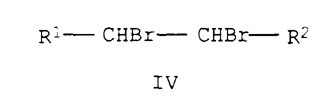 in an sich bekannter Weise in einem inerten organischen Lösungsmittel in Gegenwart einer Base umsetzt.

4. Verfahren zur Bekämpfung von Insekten und Akaridien, dadurch gekennzeichnet, daß man die Insekten und Akaridien und/oder ihren Lebensraum mit einer wirksamen Menge eines Acetylenderivats der allgemeinen Formel IA
R¹-C≡C-R² Ia
in der die Substituenten folgende Bedeutung haben:
R¹ 2-Thienyl oder 3-Thienyl, welches ein bis drei der folgenden Reste tragen kann: Nitro, Halogen, C₁-C₄-Alkyl, C₁-C₄-Halogenalkyl, C₁-C₄-Alkoxy, C₁-C₄-Halogenalkylthio, C₃-C₆-Alkenyl oder C₃-C₈-Cycloalkyl;
R² Phenyl, 1-Naphthyl oder 2-Naphthyl, welches ein bis fünf Halogenatome und/oder ein bis drei der folgenden Gruppen tragen kann: Nitro, Cyano, C₁-C₆-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₃-C₆-Alkenyl, Phenyl oder Phenoxy, wobei die letztgenannten aromatischen Gruppen ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio,
behandelt.

## Claims (Claims for the following Contracting State(s): BE, CH, DE, DK, FR, GB, IT, LI, NL)

1. An acetylene derivative of the formula I
R¹-C≡C-R² I
where
R¹ is 2-thienyl or 3-thienyl which may carry from one to three of the following radicals: nitro, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-C₆-alkenyl or C₃-C₈-cycloalkyl,
R² is phenyl, 1-naphthyl or 2-naphthyl which may carry from one to five halogen atoms and/or from one to three of the following groups: nitro, cyano, C₁-C₆-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxycarbonyl, C₃-C₆-alkenyl, phenyl or phenoxy, where the last-mentioned aromatic groups may in turn carry from one to five halogen atoms and/or from one to three of the following groups: C₁-C₄-alkyl, C₁-C₄-haloalkyl, C₁-C₄-alkoxy, C₁-C₄-haloalkoxy, C₁-C₄-alkylthio or C₁-C₄-haloalkylthio, and R¹ is not 2-thienyl when R² is phenyl or 4-methylphenyl, R¹ is not 2-thienyl, 5-chloro-2-thienyl, 3,5-dichloro-2-thienyl, 3-methyl-2-thienyl, 5-methyl-2-thienyl, 5-methoxy-2-thienyl, 3-thienyl, 2,5-dichloro-3-thienyl, 2-methyl-3-thienyl or 5-chloro-3-thienyl when R² is 4-C₁-haloalkylphenyl, R¹ is not 2-thienyl when R² is 2-X-4-C₁-haloalkylphenyl where X is fluorine, chlorine, methyl, ethyl, isopropyl, n-butyl, trifluoromethyl, methoxy or ethoxy, R¹ is not 5-chloro-2-thienyl when R² is 2-X-4-C₁-haloalkylphenyl where X is fluorine, chlorine, methyl or methoxy, R¹ is not 3-thienyl when R² is 2-X-4-C₁-haloalkylphenyl where X is fluorine, chlorine, methyl, methoxy or trifluoromethyl, R¹ is not 3-methyl-2-thienyl, 2,5-dichloro-3-thienyl or 2-methyl-3-thienyl when R² is 2-X-4-C₁-haloalkylphenyl where X is fluorine or chlorine, R¹ is not 3-bromo-5-methyl-2-thienyl when R² is phenyl, R¹ is not 5-methyl-2-thienyl when R² is phenyl, and R¹ is not 3-thienyl when R² is phenyl.

2. A process for the preparation of a compound of the formula I as claimed in claim 1, wherein an alkyne of the formula IIa or IIb 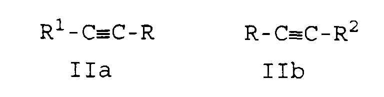 where R is hydrogen or a (C₁-C₃-alkyl)₂C(OH) protective group, is reacted with an aryl halide of the formula IIIa or IIIb 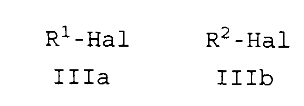 where Hal is halogen, in a conventional manner in an inert organic solvent in the presence of a base and of a palladium and copper catalyst.

3. A process for the preparation of a compound of the formula I as claimed in claim 1, wherein a dibromoethane of the formula IV 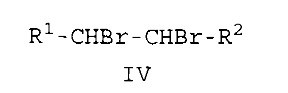 is converted in a conventional manner in an inert organic solvent in the presence of a base.

4. An insecticide or acaricide containing an effective amount of an acetylene derivative of the formula I as claimed in claim 1 and inert additives.

5. A method for controlling insects and acaridae, wherein the insects and acaridae and/or their habitat are or is treated with an effective amount of an acetylene derivative of the formula IA
R¹-C≡C-R² IA
where
R¹ is 2-thienyl or 3-thienyl which may carry from one to three of the following radicals: nitro, halogen, C₁-C₄-alkyl, C₁-C₄-haloalkyl, C₁-C₄-alkoxy, C₁-C₄-haloalkylthio, C₃-C₆-alkenyl or C₃-C₈-cycloalkyl,
R² is phenyl, 1-naphthyl or 2-naphthyl which may carry from one to five halogen atoms and/or from one to three of the following groups: nitro, cyano, C₁-C₆-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxycarbonyl, C₃-C₆-alkenyl, phenyl or phenoxy, where the last-mentioned aromatic groups may in turn carry from -one to five halogen atoms and/or from one to three of the following groups: C₁-C₄-alkyl, C₁-C₄-haloalkyl, C₁-C₄-alkoxy, C₁-C₄-haloalkoxy, C₁-C₄-alkylthio or C₁-C₄-haloalkylthio.

## Claims (Claims for the following Contracting State(s): ES)

1. An insecticide or acaricide containing an effective amount of an acetylene derivative of the formula I
R¹-C≡C-R² I
where
R¹ is 2-thienyl or 3-thienyl which may carry from one to three of the following radicals: nitro, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-C₆-alkenyl or C₃-C₈-cycloalkyl,
R² is phenyl, 1-naphthyl or 2-naphthyl which may carry from one to five halogen atoms and/or from one to three of the following groups: nitro, cyano, C₁-C₆-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxycarbonyl, C₃-C₆-alkenyl, phenyl or phenoxy, where the last-mentioned aromatic groups may in turn carry from one to five halogen atoms and/or from one to three of the following groups: C₁-C₄-alkyl, C₁-C₄-haloalkyl, C₁-C₄-alkoxy, C₁-C₄-haloalkoxy, C₁-C₄-alkylthio or C₁-C₄-haloalkylthio, and R¹ is not 2-thienyl when R² is phenyl or 4-methylphenyl, R¹ is not 2-thienyl, 5-chloro-2-thienyl, 3,5-dichloro-2-thienyl, 3-methyl-2-thienyl, 5-methyl-2-thienyl, 5-methoxy-2-thienyl, 3-thienyl, 2,5-dichloro-3-thienyl, 2-methyl-3-thienyl or 5-chloro-3-thienyl when R² is 4-C₁-haloalkylphenyl, R¹ is not 2-thienyl when R² is 2-X-4-C₁-haloalkylphenyl where X is fluorine, chlorine, methyl, ethyl, isopropyl, n-butyl, trifluoromethyl, methoxy or ethoxy, R¹ is not 5-chloro-2-thienyl when R² is 2-X-4-C₁-haloalkylphenyl where X is fluorine, chlorine, methyl or methoxy, R¹ is not 3-thienyl when R² is 2-X-4-C₁-haloalkylphenyl where X is fluorine, chlorine, methyl, methoxy or trifluoromethyl, R¹ is not 3-methyl-2-thienyl, 2,5-dichloro-3-thienyl or 2-methyl-3-thienyl when R² is 2-X-4-C₁-haloalkylphenyl where X is fluorine or chlorine, R¹ is not 3-bromo-5-methyl-2-thienyl when R² is phenyl, R¹ is not 5-methyl-2-thienyl when R² is phenyl, and R¹ is not 3-thienyl when R² is phenyl,
and inert additives.

2. A process for the preparation of a compound of the formula I as claimed in claim 1, wherein an alkyne of the formula IIa or IIb 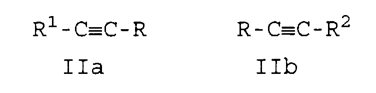 where R is hydrogen or a (C₁-C₃-alkyl)₂C(OH) protective group, is reacted with an aryl halide of the formula IIIa or IIIb 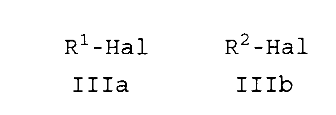 where Hal is halogen, in a conventional manner in an inert organic solvent in the presence of a base and of a palladium and copper catalyst.

3. A process for the preparation of a compound of the formula I as claimed in claim 1, wherein a dibromoethane of the formula IV 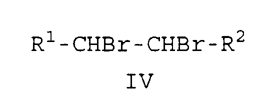 is converted in a conventional manner in an inert organic solvent in the presence of a base.

4. A method for controlling insects and acaridae, wherein the insects and acaridae and/or their habitat are or is treated with an effective amount of an acetylene derivative of the formula IA
R¹-C≡C-R² IA
where
R¹ is 2-thienyl or 3-thienyl which may carry from one to three of the following radicals: nitro, halogen, C₁-C₄-alkyl, C₁-C₄-haloalkyl, C₁-C₄-alkoxy, C₁-C₄-haloalkylthio, C₃-C₆-alkenyl or C₃-C₈-cycloalkyl,
R² is phenyl, 1-naphthyl or 2-naphthyl which may carry from one to five halogen atoms and/or from one to three of the following groups: nitro, cyano, C₁-C₆-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxycarbonyl, C₃-C₆-alkenyl, phenyl or phenoxy, where the last-mentioned aromatic groups may in turn carry from one to five halogen atoms and/or from one to three of the following groups: C₁-C₄-alkyl, C₁-C₄-haloalkyl, C₁-C₄-alkoxy, C₁-C₄-haloalkoxy, C₁-C₄-alkylthio or C₁-C₄-haloalkylthio.

## Revendications (Revendications pour l'(les) Etat(s) contractant(s) suivant(s): BE, CH, DE, DK,FR, GB, IT, LI, NL)

1. Dérivés acétyléniques de formule générale I
R¹-C≡C-R² I
dans laquelle les symboles ont les significations suivantes :
R¹ représente un groupe 2-thiényle ou 3-thiényle qui peut porter un à trois des substituants suivants : nitro, halogéno, alkyle en C1-C4, halogénoalkyle en C1-C4, alcoxy en C1-C4, halogénoalcoxy en C1-C4, alkylthio en C1-C4, halogénoalkylthio en C1-C4, alcényle en C3-C6 ou cycloalkyle en C3-C8 ;
R² représente un groupe phényle, 1-naphtyle ou 2-naphtyle, qui peut porter un à cinq atomes d'halogènes et/ou un à trois des substituants suivants : nitro, cyano, alkyle en C1-C6, halogénoalkyle en C1-C4, alcoxy en C1-C4, halogénoalcoxy en C1-C4, alkylthio en C1-C4, halogénoalkylthio en C1-C4, (alcoxy en C1-C4)carbonyle, alcényle en C3-C6, phényle ou phénoxy, ces derniers groupes aromatiques pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4,
sous réserve que R¹ ne peut représenter un groupe 2-thiényle lorsque R² représente un groupe phényle ou 4-méthylphényle, R¹ ne peut représenter un groupe 2-thiényle, 5-chloro-2-thiényle, 3,5-dichloro-2-thiényle, 3-méthyl-2-thiényle, 5-méthyl-2-phényle, 5-méthoxy-2-thiényle, 3-thiényle, 2,5-dichloro-3-thiényle, 2-méthyl-3-thiényle, 5-chloro-3-thiényle lorsque R² représente un groupe 4-C1-halogénoalkylphényle, R¹ ne peut représenter un groupe 2-thiényle lorsque R² représente un groupe 2-X-4-Cl-halogénoalkylphényle, X représentant le fluor, le chlore, un groupe méthyle, éthyle, isopropyle, n-butyle, trifluorométhyle, méthoxy ou éthoxy, R¹ ne peut représenter un groupe 5-chloro-2-thiényle lorsque R² représente un groupe 2-X-4-Cl-halogénoalkylphényle, X représentant le fluor, le chlore, un groupe méthyle ou méthoxy, R¹ ne peut représenter un groupe 3-thiényle lorsque R² représente un groupe 2-X-4-C1-halogénoalkylphényle, X représentant le fluor, le chlore, un groupe méthyle, méthoxy ou trifluorométhyle, R¹ ne peut représenter un groupe 3-méthyl-2-thiényle, 2,5-dichloro-3-thiényle ou 2-méthyl-3-thiényle lorsque R² représente un groupe 2-X-4-C1-halogénoalkylphényle, X représentant le fluor ou le chlore, R¹ ne peut représenter un groupe 3-bromo-5-méthyl-2-thiényle lorsque R² représente un groupe phényle, R¹ ne peut représenter un groupe 5-méthyl-2-thiényle lorsque R² représente un groupe phényle, et R¹ ne peut représenter un groupe 3-thiényle lorsque R² représente un groupe phényle.

2. Procédé de préparation des composés de formule I selon la revendication 1, caractérisé par le fait que l'on fait réagir un alcyne de formule générale IIa ou IIb 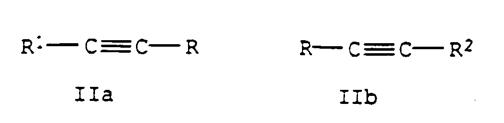 dans lesquelles R représente l'hydrogène ou un groupe protecteur (alkyle en C1-C3)₂C(OH), de manière connue en soi, dans un solvant organique inerte, en présence d'une base et d'un catalyseur au palladium et au cuivre, avec un halogénure d'aryle de formule générale IIIa ou IIIb 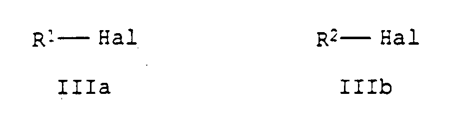 dans lesquelles Hal représente un atome d'halogène.

3. Procédé de préparation des composés de formule I selon la revendication 1, caractérisé par le fait que l'on fait réagir un dibrométhane de formule générale IV 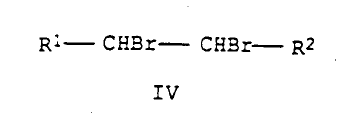 de manière connue en soi, dans un solvant organique inerte, en présence d'une base.

4. Produit pour la lutte contre les insectes et les acariens, contenant une quantité efficace d'un dérivé acétylénique de formule générale I selon la revendication 1 et des additifs inertes.

5. Procédé pour combattre les insectes et les acariens, caractérisé par le fait que l'on traite les insectes et acariens et/ou leur habitat par une quantité efficace d'un dérivé acétylénique de formule générale IA
R¹-C≡C-R² IA
dans laquelle les symboles ont les significations suivantes :
R¹ : un groupe 2-thiényle ou 3-thiényle qui peut porter un à trois des substituants suivants : nitro, halogéno, alkyle en C1-C4, halogénoalkyle en C1-C4, alcoxy en C1-C4, halogénoalkylthio en C1-C4, alcényle en C3-C6 ou cycloalkyle en C3-C8 ;
R² : un groupe phényle, 1-naphtyle ou 2-naphtyle qui peut porter un à cinq atomes d'halogènes et/ou un à trois des substituants suivants : nitro, cyano, alkyle en C1-C6, halogénoalkyle en C1-C4, alcoxy en C1-C4, halogénoalcoxy en C1-C4, alkylthio en C1-C4, halogénoalkylthio en C1-C4, (alcoxy en C1-C4)carbonyle, alcényle en C3-C6, phényle ou phénoxy, les groupes aromatiques mentionnés en dernier pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4.

## Revendications (Revendications pour l'(les) Etat(s) contractant(s) suivant(s): ES)

1. Produit pour combattre les insectes et les acariens, contenant une quantité efficace d'un dérivé acétylénique de formule générale I
R¹-C≡C-R² I
dans laquelle les symboles ont les significations suivantes :
R¹ représente un groupe 2-thiényle ou 3-thiényle qui peut porter un à trois des substituants suivants : nitro, halogéno, alkyle en C1-C4, halogénoalkyle en C1-C4, alcoxy en C1-C4, halogénoalcoxy en C1-C4, alkylthio en C1-C4, halogénoalkylthio en C1-C4, alcényle en C3-C6 ou cycloalkyle en C3-C8 ;
R² représente un groupe phényle, 1-naphtyle ou 2-naphtyle, qui peut porter un à cinq atomes d'halogènes et/ou un à trois des substituants suivants : nitro, cyano, alkyle en C1-C6, halogénoalkyle en C1-C4, alcoxy en C1-C4, halogénoalcoxy en C1-C4, alkylthio en C1-C4, halogénoalkylthio en C1-C4, (alcoxy en C1-C4)carbonyle, alcényle en C3-C6, phényle ou phénoxy, ces derniers groupes aromatiques pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4,
sous réserve que R¹ ne peut représenter un groupe 2-thiényle lorsque R² représente un groupe phényle ou 4-méthylphényle, R¹ ne peut représenter un groupe 2-thiényle, 5-chloro-2-thiényle, 3,5-dichloro-2-thiényle, 3-méthyl-2-thiényle, 5-méthyl-2-phényle, 5-méthoxy-2-thiényle, 3-thiényle, 2,5-dichloro-3-thiényle, 2-méthyl-3-thiényle, 5-chloro-3-thiényle lorsque R² représente un groupe 4-C1-halogénoalkylphényle, R¹ ne peut représenter un groupe 2-thiényle lorsque R² représente un groupe 2-X-4-C1-halogénoalkylphényle, X représentant le fluor, le chlore, un groupe méthyle, éthyle, isopropyle, n-butyle, trifluorométhyle, méthoxy ou éthoxy, R¹ ne peut représenter un groupe 5-chloro-2-thiényle lorsque R² représente un groupe 2-X-4-Cl-halogénoalkylphényle, X représentant le fluor, le chlore, un groupe méthyle ou méthoxy, R¹ ne peut représenter un groupe 3-thiényle lorsque R² représente un groupe 2-X-4-C1-halogénoalkylphényle, X représentant le fluor, le chlore, un groupe méthyle, méthoxy ou trifluorométhyle, R¹ ne peut représenter un groupe 3-méthyl-2-thiényle, 2,5-dichloro-3-thiényle ou 2-méthyl-3-thiényle lorsque R² représente un groupe 2-X-4-C1-halogénoalkylphényle, X représentant le fluor ou le chlore, R¹ ne peut représenter un groupe 3-bromo-5-méthyl-2-thiényle lorsque R² représente un groupe phényle, R¹ ne peut représenter un groupe 5-méthyl-2-thiényle lorsque R² représente un groupe phényle, et R¹ ne peut représenter un groupe 3-thiényle lorsque R² représente un groupe phényle, et des additifs inertes.

2. Procédé de préparation des composés de formule I selon la revendication 1, caractérisé par le fait que l'on fait réagir un alcyne de formule générale IIa ou IIb 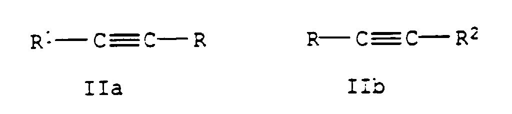 dans lesquelles R représente l'hydrogène ou un groupe protecteur (alkyle en C1-C3)₂C(OH), de manière connue en soi, dans un solvant organique inerte, en présence d'une base et d'un catalyseur au palladium et au cuivre, avec un halogénure d'aryle de formule générale IIIa ou IIIb 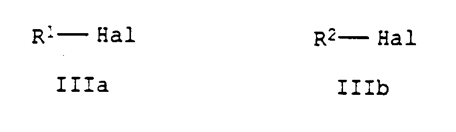 dans lesquelles Hal représente un atome d'halogène.

3. Procédé de préparation des composés de formule I selon la revendication 1, caractérisé par le fait que l'on fait réagir un dibrométhane de formule générale IV 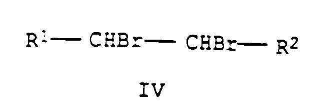 de manière connue en soi, dans un solvant organique inerte, en présence d'une base.

4. Procédé pour combattre les insectes et les acariens, caractérisé par le fait que l'on traite les insectes et acariens et/ou leur habitat par une quantité efficace d'un dérivé acétylénique de formule générale IA
R¹-C≡C-R² IA
dans laquelle les symboles ont les significations suivantes :
R¹ : un groupe 2-thiényle ou 3-thiényle qui peut porter un à trois des substituants suivants : nitro, halogéno, alkyle en C1-C4, halogénoalkyle en C1-C4, alcoxy en C1-C4, halogénoalkylthio en C1-C4, alcényle en C3-C6 ou cycloalkyle en C3-C8 ;
R² : un groupe phényle, 1-naphtyle ou 2-naphtyle qui peut porter un à cinq atomes d'halogènes et/ou un à trois des substituants suivants : nitro, cyano, alkyle en C1-C6, halogénoalkyle en C1-C4, alcoxy en C1-C4, halogénoalcoxy en C1-C4, alkylthio en C1-C4, halogénoalkylthio en C1-C4, (alcoxy en C1-C4)carbonyle, alcényle en C3-C6, phényle ou phénoxy, les groupes aromatiques mentionnés en dernier pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4.
